# EUROPEAN PATENT SPECIFICATION

(11) **EP 1 443 948 B1**
(45) Date of publication and mention of the grant of the patent: **29.06.2005**
(21) Application number: 02765096.9
(22) Date of filing: 04.10.2002
(51) Int. Cl.: A61K 35/78, A61K 31/70, A61K 9/14, A61P 9/00

(54) **PROCESS FOR THE PREPARATION OF ANTHOCYANIN-CONTAINING PRODUCTS**
VERFAHREN ZUR HERSTELLUNG VON ANTHOCYANIN-ENTHALTENDEN PRODUKTEN
PROCEDE POUR LA PREPARATION DES PRODUITS RENFERMANT DES ANTHOCYANINES

(30) Priority: 09.11.2001 GB 0127031
(43) Date of publication of application: 11.08.2004
(73) Proprietor: Medpalett Pharmaceuticals AS, 4327 Sandnes (NO)
(72) Inventor: SLIMESTAD, Rune c/o Medpalett Pharmaceuticals AS, N-4327 Sandnes (NO)
(74) Representative: Cockbain, Julian, Dr.
(86) International application number: PCT/GB2002/004506
(87) International publication number: WO 2003/039569

(56) References cited:
- EP-A- 0 412 300
- EP-A- 0 573 777
- WO-A-84/03216

## Description

The present invention relates to a process for preparing a nutritional supplement (nutraceutical) comprising a mixture of anthocyanins.

Anthocyanins are glycosides of flavylium salts. Each anthocyanin thus comprises three component parts: the hydroxylated core (the aglycone); the saccharide unit; and the counterion. Anthocyanins are naturally occurring pigments present in many flowers and fruit and individual anthocyanins are available commercially as the chloride salts, e.g. from Polyphenols Laboratories AS, Sandnes, Norway.

As individual compounds, anthocyanins have been proposed for use as antioxidants (e.g. as free radical scavengers) for treatment of the vascular system.

We have found that the beneficial effects of individual anthocyanins are enhanced if instead of an individual anthocyanin, a combination of different anthocyanins is administered orally, in particular a combination comprising both mono and disaccharide anthocyanins. It is thought that the synergistic effect arises at least in part from the different solubilities and different uptake profiles of the different anthocyanins.

However, while the combination of different anthocyanins is central to the present invention, it also represents a problem to produce due to their different physicochemical properties. The commercially available individual anthocyanins do not readily mix to form a free-flowing powder such as would be particularly suitable for tableting or filling into capsules. Normal fruit extracts containing anthocyanins cannot be spray-dried to a free-flowing powder yielding instead a sticky mess. We have found that for spray drying to be successful, the anthocyanin solution must be freed of free sugars and acids, and plant-derived lipids and macromolecules, in particular lectins.

Thus viewed from one aspect the invention provides a process for the production of an anthocyanin-containing product, said process comprising: loading an aqueous anthocyanin solution (e.g. a liquid plant extract) onto a cation exchange resin; flushing the resin with water; applying an acidic alkanol eluant to said resin until anthocyanin begins to elute therefrom whereafter applying a non-acidic alkanol eluant to said resin; collecting anthocyanin-containing eluate from said resin; evaporating off alkanol from said eluate; adding water and if necessary an anticoagulant to the eluant to produce an aqueous anthocyanin solution, preferably a solution containing at least two anthocyanins; spray-drying said solution; and optionally coating the spray dried product (optionally after mixing with further physiologically tolerable components, e.g. vitamin C, further anthocyanins, etc. and/or after tableting).

The product of the process of the invention is preferably an orally ingestible composition, e.g. a nutritional supplement, comprising a coated spray-dried material comprising at least four anthocyanins, at least one of which is a monosaccharide and at least one of which is a disaccharide.

The product preferably contains at least three monosaccharide anthocyanins. Moreover it preferably contains at least one monosaccharide anthocyanin in which the saccharide is arabinose.

The product preferably contains at least one disaccharide anthocyanin in which the disaccharide is rutinose (i.e. 6-rhamnosyl-glucose).

Moreover the product preferably contains anthocyanins with at least two different aglycones, more preferably at least four.

Especially preferably the product contains anthocyanins in which the aglycone units are cyanidin, peonidin, delphinidin, petunidin, malvidin and optionally also pelargonidin.

In one preferred embodiment, the product also contains at least one trisaccharide anthocyanin.

Example of anthocyanins suitable for use in the invention include: cyanidin-3-O-β-glucoside; cyanidin-3-O-β-galactoside; cyanidin-3-O-α-arabinoside; cyanidin-3-O-β-xyloside; cyanidin-3-O-(6"-O-α-rhamnosyl-β-glucoside); cyanidin-3-O-(2"-O-β-glucosyl-β-galactoside); cyanidin-3-O-(2"-O-β-glucosyl-β-glucoside); cyanidin-3-O-(2"-O-β-xylosyl-β-glucoside); cyanidin-3-O- (2"-O-β-xylosyl-β-galactoside); cyanidlin-3,5-di-O-β-glucoside; cyanidin-3-O-β-galactoside-5-O-β-glucoside; cyanidin-3-O-α-arabinoside-5-O-β-glucoside; cyanidin-3-O-(2"-O-β-xylosyl-β-glucoside)-5-O-β-glucoside; cyanidin-3-O-(2"-O-β-xylosyl-6"-O-β-glucosyl-β-galactoside); pelargonidin-3-O-β-glucoside; pelargonidin-3-O-(6"-O-α-rhamnosyl-β-glucoside) ; pelargonidin-3,5-di-O-β-glucoside; peonidin-3-O-β-glucoside; peonidin-3-O-α-arabinoside; peonidin-3,5-di-O-β-glucoside; delphinidin-3-O-β-glucoside; delphinidin-3-O-(6"-O-α-rhamnosyl-β-glucoside); delphinidin-3-O-(2"-O-β-xylosyl-β-glucoside); petunidin-3-O-β-glucoside; malvidin-3-O-β-glucoside; malvidin-3,5-di-O-β-glucoside; and malvidin-3-O-α-arabinoside-5-O-β-glucoside.

If desired, one or more hydroxy groups, especially on the saccharide unit, in the anthocyanins may be acylated, e.g. carrying a C₁₋₁₂, more especially a C₃₋₉ saturated or unsaturated acyl group, for example a mono- or dicarboxylic acid residue, e.g. a malonyl, p-coumaryl or feruloylyl group. Thus for example such acylated compounds include cyanidin-3-O-(6"-O-(E-p-coumaryl)-2"-O-β-xylosyl-β-glucoside); cyanidin-3-O-(6"-O-(E-p-coumaryl) -2"-O-β-xylosyl-β-glucoside)-5-O-β-glucoside; cyanidin-3-O-(2"-O-β-xylosyl-6"-O-(E-feruloyl-β-glucosyl)-β-galactoside); cyanidin-3-O-(2"-O-β-xylosyl-6"-O-[E-coumaryl-β-glucosyl]-β-galactoside); and petunidin-3-O-(6"-O-(4"'-O-E-coumaryl)-α-rhamnosyl-β-glucoside)-5-O-β-glucoside.

The counterion in the anthocyanins in the product of the invention may be any physiologically tolerable counteranions, e.g. chloride, succinate, fumarate, malate, maleate, citrate, etc. Preferably however the counterion is a fruit acid anion, in particular citrate, as this results in the products having a particularly pleasant taste.

Particularly suitable sources for the anthocyanins are fruits such as cherries, bilberries, blueberries, blackcurrants, redcurrants, grapes, cranberries, strawberries, and apples and vegetables such as red cabbage. Bilberries, in particular Vaccinium myrtillus, and blackcurrants, in particular Ribes nigrum, are especially suitable. The berries of V. myrtillus contain fifteen monosaccharide anthocyanins, namely the aglycone:saccharide combinations of cyanidin, peonidin, delphinidin, petunidin and malvidin and glucose, galactose and arabinose. The currants of R. nigrum contain four anthocyanins, namely the 3-glucosides and 3-rutinosides of cyanidin and delphinidin.

The disaccharide anthocyanins are more watersoluble than the monosaccharides; moreover cyanidin and delphinidin anthocyanins are amongst the most watersoluble anthocyanins.

Besides the anthocyanins, the products of the process of the invention may desirably contain further beneficial or inactive ingredients, e.g. vitamins (in particular vitamin C), flavones, isoflavones, anticoagulants (e.g. maltodextrin, silica, etc.), desiccants, etc. Desirably however the anthocyanins constitute at least 50% by weight of the product compositions, excluding the coating material.

In the process of the invention, spray-drying is preferably effected using by spraying into an inert atmosphere, e.g. a nitrogen atmosphere, with inlet temperatures of 130 to 160°C and flow rates of 5 to 12 L/hour. If the spray-dried product is sticky, then increasing flow rate and inlet temperature and/or increasing anticoagulant content and/or increasing atomiser rotation rate will result in a non-sticky product. The optimum temperature, flow-rate, etc. can be determined in this way for each separate anthocyanin source. The anthocyanin solution to be spray-dried desirably is an aqueous solution containing anthocyanins at 5 to 15% wt, more preferably 8 to 12% wt, dry solids basis.

The spray-dried product is preferably coated. This is important due to the hygroscopic nature of the anthocyanins. Coating may be by conventional coating techniques following tableting of the spray dried product; alternatively, and preferably, coating is achieved by filling the spray dried product into capsules. While conventional gelatin capsules may be used, it is preferred to use cellulose capsules, such as Vcaps from Capsugel SA, Belgium.

The coated compositions preferably contain 50 to 250 mg anthocyanin per dose unit (e.g. capsule), more preferably 70 to 160 mg.

The dosage for a human consumer is preferably 50 to 250 mg anthocyanin per day, e.g. one or two 75 mg anthocyanin capsules per day. This corresponds approximately to the anthocyanin content of one or two cups full of fresh berries.

As mentioned above, the product compositions preferably also contain vitamin C, e.g. 10 to 1000, preferably 20 to 200, mg per gram anthocyanin.

Besides anthocyanins, the products will generally also contain a anticoagulant, e.g. maltodextrin, lactose or silica, added to the anthocyanin solution to be spray dried to prevent coagulation in the spray drying process. Typically the anticoagulant will be present at 0.3 to 0.8g, especially 0.5g, per gram anthocyanin.

Where anthocyanins from more than one plant source are to be included in the products, they are preferably mixed after spray-drying. Thus, for example, the product compositions preferably contain anthocyanins from V. myrtillus and R. nigrum mixed after spray-drying, preferably in a weight ratio of 0.5:1 to 1:0.5, especially 1:1.

As mentioned above, the individual anthocyanins available commercially and used in research and as the active components of medicaments are not readily formulated into the sorts of free-flowing powders that are particularly suitable for tableting or capsule filling. This is inconvenient for handling and formulation, and the spray drying procedure of the invention may readily be used to transform such compounds into an easily handleable free-flowing powder. This forms a further aspect of the invention.

The invention will now be described further with reference to the following Examples.

### EXAMPLE 1

### Anthocyanin extraction

As raw material is used the cakes of fruit skin produced as the waste product in fruit juice pressing from V. myrtillus and R. nigrum. Fruit juice producers generally add lectinases to the fruit before pressing to release anthocyanins into the juice produced; however the cake still contains a high anthocyanin content.

200L methanol, with 0.1% wt. HCl content, is added to 120 kg fruit skin cake. The mixture is allowed to stand for 24 hours at ambient temperature (20-25°C) whereafter the methanol is drained off. 150L methanol (0.1% HCl) is then added to the fruit skin. The mixture is allowed to stand for 24 hours at ambient temperature (20-25°C) whereafter the methanol is drained off. A further 150L methanol (0.1% HCl) is then added to the fruit skin. The mixture is allowed to stand for 24 hours at ambient temperature (20-25°C) whereafter the methanol is drained off. The methanolic solutions are combined and concentrated to 30% on a nanofilter membrane (e.g. such as are available from Osmonics) with an exclusion limit of 300 D.

If desired, ethanol may be used in place of methanol and a lectinase may be added to the methanol for the soaking step.

The concentrated methanolic solutions are further concentrated to 5% wt. dry solids content on a rotary evaporator.

Ion-exchanged water (5 to 10L) is added to the concentrate to produce an essentially aqueous solution which is then mixed thoroughly in about 1:1 volume ratio with ethyl acetate. The phases are separated and traces of ethyl acetate are evaporated from the anthocyanin containing aqueous phase.

The aqueous phase is then loaded onto an ion exchange column (e.g. Amberlite XAD7) which is flushed with ion exchanged water.

Methanol, 0.25% wt. citric acid, is then added to the column until anthocyanins begin to appear in the eluate whereafter elution is continued with pure methanol. In this way the eluate collected is essentially free of free citric acid.

The eluate is evaporated down on a rotary evaporator to a dry solids content of 10% wt. Water is added and the remaining methanol is evaporated off to a dry solids content of 10% wt. Maltodextrin is then added to bring the dry solids content up to 15% wt.

### EXAMPLE 2

### Spray-drying

The aqueous solution produced in Example 1 is spray-dried in a pharmaceutical spray dryer (Type SD-4-R-CC) from Niro A/S, Copenhagen, Denmark in a nitrogen atmosphere, using a rotary atomizer, with an inlet temperature of 130-160°C and with a flow rate of 5 to 12 L/hour.

The product is a free flowing deep purple powder which should be stored in a dry atmosphere.

### EXAMPLE 3

### Capsules

The spray-dried products from V. myrtillus and R. nigrum, produced in Example 2 (with a water content of less than 5% wt.) are mixed in a 1:1 weight ratio and filled into cellulose capsules (V caps) to produce an anthocyanin content per capsule of 75 mg or 150 mg.

The capsules are then packed in blister packs.

## Claims

1. A process for the production of an anthocyanin-containing product, said process comprising: loading an aqueous anthocyanin solution onto a cation exchange resin; flushing the resin with water; applying an acidic alkanol eluant to said resin until anthocyanin begins to elute therefrom whereafter applying a non-acidic alkanol eluant to said resin; collecting anthocyanin-containing eluate from said resin; evaporating off alkanol from said eluate adding water and if necessary an anticoagulant to the eluate to produce an aqueous anthocyanin solution, preferably a solution containing at least two anthocyanins; spray-drying said solution; and optionally coating the spray dried product.

2. A process as claimed in claim 1 wherein the spray-dried product is mixed with further anthocyanins and coated.

3. A process as claimed in either of claims 1 and 2 wherein said aqueous anthocyanin solution contains at least one anthocyanin selected from cyanidin-3-O-β-glucoside; cyanidin-3-O-β-galactoside; cyanidin-3-O-α-arabinoside; cyanidin-3-O-β-xyloside; cyanidin-3-O-(6"-O-α-rhamnosyl-β-glucoside); cyanidin-3-O-(2"-O-β-glucosyl-β-galactoside); cyanidin-3-O-(2"-O-β-glucosyl-β-glucoside); cyanidin-3-O-(2"-O-β-xylosyl-β-glucoside); cyanidin-3-O-(2"-O-β-xylosyl-β-galactoside); cyanidin-3,5-di-O-β-glucoside; cyanidin-3-O-β-galactoside-5-O-β-glucoside; cyanidin-3-O-α-arabinoside-5-O-β-glucoside; cyanidin-3-O-(2"-O-β-xylosyl-β-glucoside)-5-O-β-glucoside; cyanidin-3-O-(2"-O-β-xylosyl-6"-O-β-glucosyl-β-galactoside); pelargonidin-3-O-β-glucoside; pelargonidin-3-O-(6"-O-α-rhamnosyl-β-glucoside); pelargonidin-3,5-di-O-β-glucoside; peonidin-3-O-β-glucoside; peonidin-3-O-α-arabinoside; peonidin-3,5-di-O-β-glucoside; delphinidin-3-O-β-glucoside; delphinidin-3-O-(6"-O-α-rhamnosyl-β-glucoside); delphinidin-3-O-(2"-O-β-xylosyl-β-glucoside); petunidin-3-O-β-glucoside; malvidin-3-O-β-glucoside; malvidin-3,5-di-O-β-glucoside; and malvidin-3-O-α-arabinoside-5-O-β-glucoside.

4. A process as claimed in any one of claims 1 to 3 wherein said aqueous anthocyanin solution contains at least one anthocyanin selected from cyanidin-3-O-(6"-O-(E-p-coumaryl)-2"-O-β-xylosyl-β-glucoside); cyanidin-3-O-(6"-O-(E-p-coumaryl)-2"-O-β-xylosyl-β-glucoside)-5-O-β-glucoside; cyanidin-3-O-(2"-O-β-xylosyl-6"-O-(E-feruloyl-β-glucosyl)-β-galactoside); cyanidin-3-O-(2"-O-β-xylosyl-6"-O-[E-coumaxyl-β-glucosyl]-β-galactoside); and petunidin-3-O-(6"-O-(4"'-O-E-coumaryl)-α-rhamnosyl-β-glucoside)-5-O-β-glucoside.

5. A process as claimed in any one of claims 1 to 4 wherein the spray-dried product is coated to produce a product containing at least four anthocyanins, at least one of which is a monosaccharide and at least one of which is a disaccharide.

6. A process as claimed in any one of claims 1 to 5 wherein said aqueous anthocyanin solution is a liquid extract from a fruit selected from cherries, bilberries, blueberries, blackcurrants, redcurrants, grapes, cranberries, strawberries and apples.

7. A process as claimed in any one of claims 1 to 6 wherein said aqueous anthocyanin solution is a liquid extract from the fruit of Vaccinium myrtillus.

8. A process as claimed in any one of claims 1 to 7 wherein said aqueous anthocyanin solution is a liquid extract from the fruit of Ribes nigrum.

9. A process as claimed in any one of claims 1 to 8 wherein the spray-dried product is coated to produce dose units containing 70 to 160 mg anthocyanin per dose.

10. A process as claimed in any one of claims 1 to 9 wherein said aqueous anthocyanin solution is a liquid extract from anthocyanins from Vaccinium myrtillus and Ribes nigrum in a weight ratio of 0.5:1 to 1:0.5.

## Patentansprüche

1. Verfahren zur Herstellung eines Anthocyanin enthaltenden Produktes, wobei das Verfahren umfasst: Beladen eines kationischen Austauschharzes mit einer wässrigen Anthocyanin-Lösung; Spülen des Harzes mit Wasser; Aufgeben eines sauren Alkanol-Eluenten auf das Harz bis das Anthocyanin anfängt hieraus zu eluieren und danach Aufgeben eines nicht-sauren Alkanol-Eluenten auf das Harz; Sammeln der Anthocyanin enthaltenden Eluate aus dem Harz; Abdampfen des Alkanols aus dem Eluat, Zugegeben von Wasser und, sofern notwendig, eines Antikoagulans zum Eluat zur Herstellung einer wässrigen Anthocyanin-Lösung, vorzugsweise einer Lösung, die wenigstens zwei Anthocyanine enthält; Sprühtrocknen der Lösung; und wahlweise Beschichten des sprühgetrockneten Produktes.

2. Verfahren nach Anspruch 1, bei dem das sprühgetrocknete Produkt mit weiteren Anthocyaninen gemischt und beschichtet wird.

3. Verfahren nach Anspruch 1 oder Anspruch 2, bei dem die wässrige Anthocyanin-Lösung wenigstens ein Anthocyanin ausgewählt aus Cyanidin-3-O-β-glucosin; Cyanidin-3-O-β-galactosid; Cyanidin-3-O-α-arabinosid; Cyanidin-3-O-β-xylosid; Cyanidin-3-O-(6"-O-α-rhamnosyl-β-glucosid); Cyanidin-3-O-(2"-O-β-glucosyl-β-galactosid); Cyanidin-3-O-(2"-β-glucosyl-β-glucosid); Cyanidin-3-O-(2"-O-β-xylosyl-β-galactosid); Cyanidin-3,5-di-O-β-glucosid; Cyanidin-3-O-β-galactosid-5-O-β-glucosid; Cyanidin-3-O-α-arabinosid-5-O-β-glucosid; Cyanidin-3-O-(2"-O-β-glucosid)-5-O-β-glucosid; Cyanidin-3-O-(2"-O-β-xylosyl-6"-O-β-glucosyl-β-galactosid); Pelargonidin-3-O-β-glucosid; Pelargonidin-3-O-(6"-O-α-rhamnosyl-β-glucosid); Pelargonidin-3,5-di-O-β-glucosid; Peonidin-3-O-β-glucosid; Peonidin-3-O-α-arabinosid; Peonidin-3,5-di-O-β-glucosid; Delphinidin-3-O-β-glucosid; Delphinidin-3-O-(6"-O-α-rhamnosyl-β-glucosid); Delphinidin-3-O-(2"-O-β-xylosyl-β-glucosid); Petunidin-3-O-β-glucosid; Malvidin-3-O-β-glucosid; Malvidin-3,5-di-O-β-glucosid; und Malvidin-3-O-α-arabinosid-5-O-β-glucosid enthält.

4. Verfahren nach einem der Ansprüche 1 bis 3, bei dem die wässrige Anthocyanin-Lösung wenigstens ein Anthocyanin ausgewählt aus Cyanidin-3-O-(6"-O-(E-p-coumaryl)-2"-O-b-xylosyl-β-glucosid); Cyanidin-3-O-(6"-O-(E-p-coumaryl)-2"-O-β-xylosyf-β-glucosid)-5-O-β-glucosid; Cyanidin-3-O-(2"-O-β-xylosyl-6"-O-(E-feruloyl-β-glucosyl)-β-galctosid); Cyanidin-3-O-(2"-O-β-xylosyl-6"-O-[E-coumaryl-β-glucosyl]-β-galactosid); und Petunidin-3-O-(6"-O-(4"'-O-E-coumaryl)-α-rhamnosyl-β-glucosid)-5-O-β-glucosid enthält.

5. Verfahren nach einem der Ansprüche 1 bis 4, bei dem das sprühgetrocknete Produkt zur Herstellung eines Produkts beschichtet wird, das wenigstens vier Anthocyanine enthält, wobei wenigstens eines derselben ein Monosaccharid und wenigstens eines derselben ein Disaccharid ist.

6. Verfahren nach einem der Ansprüche 1 bis 5, bei dem die wässrige Anthocyanin-Lösung ein Flüssigextrakt einer Frucht ausgewählt aus Kirschen, Heidelbeeren, Blaubeeren, Schwarze Johannisbeeren, Rote Johannisbeeren, Weintrauben, Moosbeeren, Erdbeeren und Äpfeln ist.

7. Verfahren nach einem der Ansprüche 1 bis 6, bei dem die wässrige Anthocyanin-Lösung ein Flüssigextrakt aus der Frucht *vaccinium myrtillus* ist.

8. Verfahren nach einem der Ansprüche 1 bis 7, bei dem die wässrige Anthocyanin-Lösung ein Flüssigextrakt aus der Frucht *ribes nigrum* ist.

9. Verfahren nach einem der Ansprüche 1 bis 8, bei dem das sprühgetrocknete Produkt zur Herstellung von Dosierungseinheiten, die 70 bis 160 mg Anthocyanin pro Dosis enthalten, beschichtet wird.

10. Verfahren nach einem der Ansprüche 1 bis 9, bei dem die wässrige Anthocyanin-Lösung ein Flüssigextrakt von Anthocyaninen aus *vaccini* *um myrtillus* und *ribes nigrum* in einem Gewichtsverhältnis von 0,5:1 bis 1:0,5 ist.

## Revendications

1. Procédé pour la production d'un produit contenant de l'anthocyanine, ledit procédé comprenant : le chargement d'une solution aqueuse d'anthocyanine sur une résine échangeuse de cations; le rinçage de la résine avec de l'eau ; l'application d'un éluant alcanol acide à ladite résine jusqu'à ce que l'anthocyanine commence à s'éluer à partir de celle-ci et ensuite l'application d'un éluant alcanol non acide à ladite résine; la collecte à partir de ladite résine de l'éluat contenant l'anthocyanine ; l'élimination par évaporation de l'alcanol à partir dudit éluat en ajoutant de l'eau et, si cela est nécessaire, un anticoagulant à l'éluat pour produire une solution aqueuse d'anthocyanine, de préférence une solution contenant au moins deux anthocyanines ; le séchage par pulvérisation de ladite solution ; et le cas échéant l'enrobage du produit séché par pulvérisation.

2. Procédé selon la revendication 1, dans lequel le produit séché par pulvérisation est mélangé avec d'autres anthocyanines et enrobé.

3. Procédé selon soit la revendication 1 soit la revendication 2, dans lequel ladite solution aqueuse d'anthocyanine contient au moins une anthocyanine choisie parmi le cyanidin-3-O-β-glucoside ; le cyanidin-3-O-β-galactoside ; le cyanidin-3-O-α-arabinoside ; le cyanidin-3-O-β-xyloside ; le cyanidin-3-O-(6"-O-α-rhamnosyl-β-glucoside) ; le cyanidin-3-O-(2"-O-β-glucosyl-β-galactoside) ; le cyanidin-3-O-(2"-O-β-glucosyl-β-glucoside); le cyanidin-3-O-(2"-O-β-xylosyl-β-glucoside) ; le cyanidin-3-O-(2"-O-β-xylosyl-β-galactoside) ; le cyanidin-3,5-di-O-β-glucoside ; le cyanidin-3-O-β-galactoside-5-O-β-glucoside ; le cyanidin-3-O-α-arabinose-5-O-β-glucoside ; le cyanidin-3-O-(2"-O-β-xylosyl-β-glucoside)-5-O-β-glucoside ; le cyanidin-3-O-(2"-O-β-xylosyl-6"-O-β-glucosyl-β-galactoside) ; le pelargonidin-3-O-β-glucoside ; le pelargonidin-3-O-(6"-O-α-rhamnosyl-β-glucoside) ; le pelargonidin-3,5-di-O-β-glucoside ; le peonidin-3-O-β-glucoside ; le peonidin-3-O-α-arabinoside ; le peonidin-3,5-di-O-β-glucoside ; le delphinidin-3-O-β-glucoside ; le delphinidin-3-O-(6"-O-α-rhamnosyl-β-glucoside) ; le delphinidin-3-O-(2"-O-β-xylosyl-β-glucoside) ; le petunidin-3-O-β-glucoside ; le malvidin-3-O-β-glucoside ; le malvidin-3,5-di-O-β-glucoside ; et le malvidin-3-O-α-arabinoside -5-O-β-glucoside.

4. Procédé selon l'une quelconque des revendications 1 à 3, dans lequel ladite solution aqueuse d'anthocyanine contient au moins une anthocyanine choisie parmi le cyanidin-3-O-(6"-O-(E-p-coumaryl)-2"-O-β-xylosyl-β-glucoside) ; le cyanidin-3-O-(6"-O-(E-p-coumaryl)-2"-O-β-xylosyl-β-glucoside)-5-O-β-glucoside ; le cyanidin-3-O-(2"-O-β-xylosyl-6"-O-(E-feruloyl-β-glucosyl)-β-galactoside) ; le cyanidin-3-O-(2"-O-β-xylosyl-6"-O-[E-coumaryl-β-glucosyl]-β-galactoside) ; et le petunidin-3-O-(6"-O-(4"'-O-E-coumaryl)-α-rhamnosyl-β-glucoside)-5-O-β-glucoside.

5. Procédé selon l'une quelconque des revendications 1 à 4, dans lequel le produit séché par pulvérisation est enrobé pour obtenir un produit contenant au moins quatre anthocyanines, dont l'une au moins est un monosaccharide et l'une au moins est un disaccharide.

6. Procédé selon l'une quelconque des revendications 1 à 5, dans lequel ladite solution aqueuse d'anthocyanine est un extrait liquide d'un fruit choisi parmi les cerises, les myrtilles, les airelles, les cassis, les groseilles, les raisins, les canneberges, les fraises et les pommes.

7. Procédé selon l'une quelconque des revendications 1 à 6, dans lequel ladite solution aqueuse d'anthocyanine est un extrait liquide du fruit de Vaccinium myrtillus.

8. Procédé selon l'une quelconque des revendications 1 à 7, dans lequel ladite solution aqueuse d'anthocyanine est un extrait liquide du fruit de Ribes nigrum.

9. Procédé selon l'une quelconque des revendications 1 à 8, dans lequel le produit séché par pulvérisation est enrobé pour produire des doses unitaires contenant 70 à 160 mg d'anthocyanine par dose.

10. Procédé selon l'une quelconque des revendications 1 à 9, dans lequel ladite solution aqueuse d'anthocyanine est un extrait liquide des anthocyanines de Vaccinium myrtillus et de Ribes nigrum selon un rapport pondéral de 0,5 : 1 à 1 : 0,5.
